Europäisches Patentamt

European Patent Office

Office européen des brevets

(19)

(11) Publication number:

**0 155 725**

**A1**

## EUROPEAN PATENT APPLICATION

(21) Application number: 85200263.3

(22) Date of filing: 26.02.85

(51) Int. Cl.⁴: **G 01 N 27/30**

(30) Priority: 27.02.84 NL 8400598
22.02.85 US 704628

(43) Date of publication of application:
25.09.85 Bulletin 85/39

(84) Designated Contracting States:
DE FR GB IT NL

(71) Applicant: CORDIS EUROPA N.V.
Oosteinde 8
NL-9301 LJ Roden(NL)

(72) Inventor: Koning, Gerrit
Drosten 56
NL-9481 GE Vries(NL)

(74) Representative: Urbanus, Henricus Maria, Ir. et al,
c/o Vereenigde Octrooibureaux Nieuwe Parklaan 107
NL-2587 BP 's-Gravenhage(NL)

(54) Ion concentration measurement system that employs measuring and reference field effect transistor electrodes sensitive to the same ion.

(57) A system is provided for measuring the ion concentration within a liquid. This electrical measuring system employs ion-sensitive field effect transistor components or transducers, known as ISFET components, both as the measuring electrode component and as the reference electrode component, each ISFET component being sensitive to the same ion, such as the hydrogen ion when the ion concentration to be measured is the pH value of the liquid. The reference ISFET electrode component is associated with a structure that provides contact between a rinsing liquid and the reference ISFET electrode component, which rinsing liquid is of a known and typically constant composition and ion concentration, or pI. Additionally, the measuring ISFET electrode component and the reference ISFET electrode component are electrically connected through a differential circuit.

FIG.2

Title: Ion Concentration Measurement System that Employs
Measuring and Reference Field Effect Transistor
Electrodes Sensitive to the Same Ion.

————

The present invention generally relates to an
ion concentration measurement system, more particularly to
a system that is capable of being incorporated into a
catheter for in vivo measuring and monitoring of the concentration of an ion within the human body. The system includes a measuring electrode and a reference electrode,
both of which are ion-sensitive field effect transistor
(ISFET) transducers that are both sensitive to the same
ion. A differential circuit connects the measuring ISFET
and the reference ISFET so that changes in one ISFET will
be measured as a real output signal and that changes in
both ISFET'S will not result in an output signal.
The reference ISFET is structured so that it is in contact
with a rinsing liquid having a known composition and a known
ion concentration.

Systems for monitoring and measuring ion concentrations have been devised and used in order to measure and
monitor ion activity within liquids such as human blood.
Some such systems incorporate an electrode that employs an
ISFET component. ISFET components are field effect transistors for the selective measurement of the concentration,
or pI, of an ion within a liquid. Often, these systems are
used in order to measure and monitor the hydrogen ion concentration, or pH, of a liquid such as blood. A variety of reference electrodes have been proposed for use in conjunction with measuring ISFET electrode components. United States Letters Patent No. 4,020,830 describes reference electrodes in this regard, while other systems employ an ion-selective electrode such as an Ag/AgCl electrode as the reference electrode which is in contact with a liquid of constant concentration. One difficulty with using an ion-selective electrode as the reference electrode is that such electrodes measure ionic concentrations and when this concentration changes such electrodes change their reference potential, which results in undesirable inconsistency and inaccuracy with respect to the entire measuring system. Ideally, a reference electrode should provide a steady reference potential within the electrochemical circuit of this type of device, particularly when the device is used for in vivo measurement and monitoring of physiologically important pI parameters.

Preferably, these types of devices are sized and structured so as to be capable of being placed in vivo within the bloodstream or the like, typically by means of a catheter structure. Such structures are superior to devices having electrodes that are adhered to the patient's skin, which is a less desirable approach in view of the unstable skin-electrode interface and their characteristic of requiring a relatively long distance between the active electrode and the reference electrode. Such devices are also difficult to apply in connection with continuous blood analysis in extra-corporal blood circuits such as those utilized during hemodialysis and open-heart surgical procedures. Accordingly, it is advantageous to carry out _in vivo_ ion concentration measurements with a system that incorporates a catheter structure in order to achieve _in vivo_ measurements within the bloodstream or the like.

Other electrical measuring systems for measuring ion concentrations include a pH-FET and a reference FET component. In some such systems both FET components are contacted with the liquid to be measured, such as the blood, one such FET component being coated with a pH-sensitive layer and the other FET component being coated with a membrane that is intended to be insensitive to any and all ions. This latter feature is not readily achieved inasmuch as it is exceedingly difficult to manufacture a membrane that is truly ion-insensitive.

The present invention provides a system for _in vivo_ ion concentration measurement and monitoring, such as that carried out within the bloodstream of a patient, which system does not experience substantial drift, and which system is designed so that both the measuring component and the reference component are sensitive to one and the same ion so that the measuring component and the reference component will exhibit substantially identical sensitivities.

In an important aspect of this invention, the measuring ISFET transducer component and the reference ISFET transducer component are connected together by means of a differential circuit so that corresponding changes in the behaviour of both ISFET'S will be compensated by the differential circuit, typical changes in behavior being the result of variations such as drift, temperature changes, aging and the like. Additionally, the reference ISFET component includes a structure so that the reference ISFET can be contacted with a rinsing liquid of known, and typically constant, composition and ion concentration. Most advantageously, the system is incorporated into a catheter structure suitable for direct incorporation into the bloodstream or the like of the patient.

It is accordingly a general object of the present invention to provide an improved ion concentration measurement system that is particularly well-suited for in vivo usage.

Another object of the present invention is to provide a device for ion concentration measurement that is especially stable with respect to inaccuracies that can develop due to drift, temperature changes, aging and the like, while also providing a device that is especially precise and sensitive to changes in the ion concentration being measured or monitored. Another object of this invention is to provide an improved ion concentration and measurement system which may be calibrated during in vivo measurements.

These and other objects, features and advantages of this invention will be clearly understood through a consideration of the following detailed description.

In the course of this description, reference will be made to the attached drawings, wherein:

Fig. 1 is an elevational view of a typical catheter structure suitable for incorporating the ion concentration measurement system in accordance with this invention;

Fig. 2 is a generally schematic longitudinal sectional view of Fig. 1;

Fig. 3 is a schematic diagram of the differential circuit by which the measuring and reference FET components are connected; and

Fig. 4 is a schematic illustration of the voltage drops that occur within the ion concentration measuring system according to this invention.

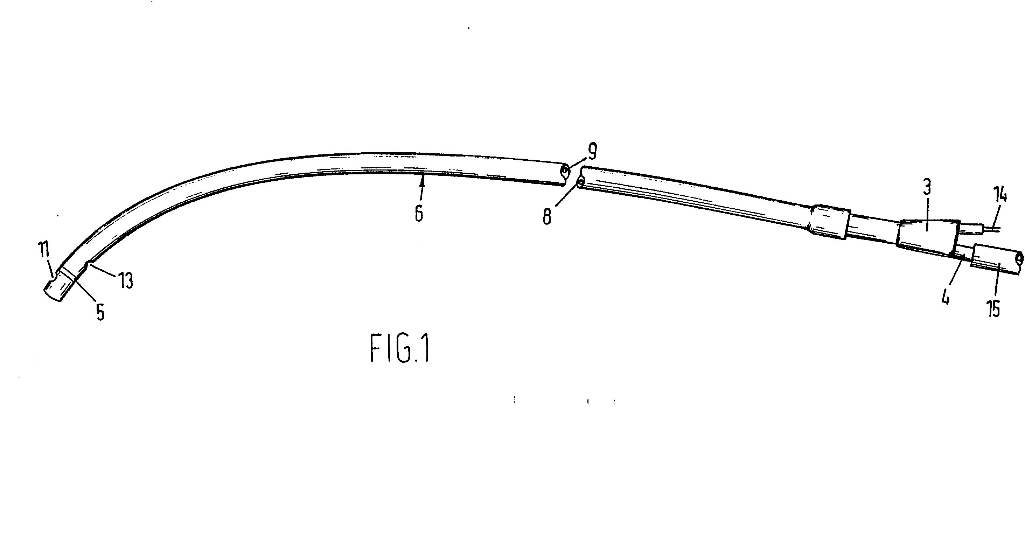

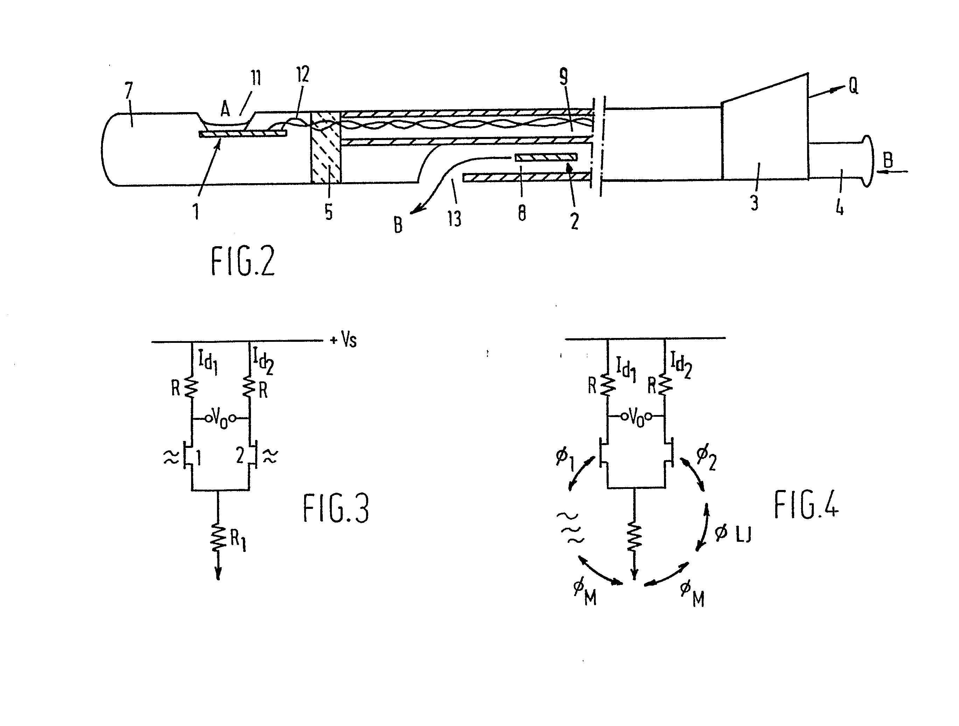

Fig. 1 shows a catheter 6 adapted for insertion into a human bloodstream, which catheter 6 has a top portion 7, within which a measuring ISFET electrode component 1 (Fig. 2) is mounted. This measuring ISFET electrode component 1 is positioned such that same will contact blood A at an opening 11 through the wall of the catheter top 7. A pair of electrical conductors 12 connect the electrical components of the measuring ISFET transducer electrode 1 to suitable known FET structures and circuitry, such connection being to the so-called drain and source locations associated with the measuring ISFET electrode component 1. Electrical conductors 12 pass through a lumen 9 that is longitudinally positioned throughout the catheter 6 in the direction of arrow Q, such being facilitated by suitable electrical contacts 14 (Fig. 1).

An annular metal electrode 5 connects the catheter top 7 to the main body portion of the catheter 6. This annular metal electrode is connected within the electrical measuring system to function as a ground for the system in accordance with known principles. This metal electrode 5 is desirable inasmuch as it provides stability as a ground within the system, but it is not necessarily essential to the operation of the measuring circuit.

A reference ISFET transducer electrode 2 is mounted within another lumen 8 of the catheter 6. The wires of electrode 2 pass through lumen 9. Lumen 8 and lumen 9 are separated from each other within the catheter 6 and are substantially longitudinally parallel with each other throughout the catheter 6 until a bifurcation 3 at a proximal location along the catheter 6. The electrical contacts 14 pass out of one branch of the bifurcation 3, while a tube 4 extends beyond the other branch of the bifurcation 3 for receiving a length tubing 15 or the like for communication to a source of rinsing liquid (not shown). The reference ISFET electrode component 2 preferably includes a chip-mounted pH-FET and an Ag/AgCl ion-selective electrode. An opening 13 is provided through the wall of the lumen 8 in the vicinity of, and preferably somewhat distally of, the reference ISFET electrode component 2. By this structure, the rinsing liquid can flow from its source, through the tube 4 and out the opening 13 in accordance with the flow B illustrated in Fig. 2. This rinsing liquid is of a constant pH and pCl. Of course the principle also works when the reference FET is sensitive to $K^+$ and the rinsing fluid has a constant $K^+$ concentration or for a $Na^+$-FET and constant $Na^+$ concentration in the rinsing fluid etc.

In use for actual measurement of the parameters of the blood A, the catheter 6 is inserted into the bloodstream such that the measuring ISFET electrode component 1 is in contact with the blood A and adjusts itself to the ion concentration, typically the pH, of the blood. At this time, the reference ISFET electrode component 2 is in contact with the rinsing liquid B and has a constant gate potential $V_{G2}$, provided the ion concentration, typically the pH of the rinsing liquid, and the liquid-junction potential are kept constant.

With the measuring ISFET electrode component 1 and the reference ISFET electrode component 2 of the measuring system being connected by a differential circuit as shown in Fig. 2, typically a bridge circuit, corresponding changes in the behavior of the measuring ISFET electrode component 1 or the reference ISFET electrode 2 will result in a corresponding change in the output of the differential circuit. Equal changes in ISFET electrode 1 and reference

-8-

electrode 2 will not give a change in output signal because of the differential nature of the circuit. Those equal changes may be due to drift, temperature changes, aging, and the like.

Components included in the ion concentration measurement system of generally known construction include an electrical voltage source for applying the drain source voltage $V_{DS}$ within each FET component 1 and 2, a voltage source for applying the gate voltage $V_{GS}$ between the source and gate via the fluid and the metal band, an amplifier system, and appropriate current conductive wiring for electrically interconnecting the various components. Details regarding these particular components will be known to those skilled in the art.

With particular reference to the voltage drop diagram of Fig. 4, the voltage $V_O$ to be measured is given by the following equation:

$$V_O = (\emptyset_1 - \emptyset_2 - \emptyset_{LJ}) \times \frac{\partial I_d}{\partial V_{GS}} \times R$$

where:

$$\frac{\partial I_d}{\partial V_{GS}} = \text{transconductance of the FET}$$

$\emptyset_{LJ}$ = liquid-junction potential = constant

$\emptyset_1 - \emptyset_2 \; \alpha \; \Delta pH = pH_{blood} - pH_{rinsing\;liquid}$

Consequently: $V_O \; \alpha \; \Delta pH$.

The actual pH value of the blood measured in accordance with this invention is obtained by adding the pH value of the rinsing liquid to the $\Delta$pH that is actually measured by the differential circuit.

If desired or necessary, an _in vivo_ calibration of the ion concentration measurement system according to this invention can be performed in a relatively simple manner. First, the reference ISFET electrode component 2, with its Ag/AgCl electrode, is exposed to the rinsing liquid, and the instrument is set to the ion concentration, typically the pH value, of the rinsing liquid. In a subsequent, second step, blood is admitted into the lumen 8 and into contact with the reference ISFET electrode component 2, and the output signal $V_o$ of the differential circuit is noted. If the $V_o$ has a value of other than zero mVolts, then the setting of the measuring ISFET electrode component 1 is adjusted until the magnitude of the output signal $V_o$ of the differential circuit is zero mVolts. Before employing the ion concentration measurement system to accomplish actual measurement or monitoring, the blood within the lumen 8 can be expelled from the lumen 8 by means of the rinsing liquid, after which the ion concentration measurement system is suitably calibrated and ready for use. By the first calibrating step, in which the reference ISFET electrode component is exposed to the rinsing liquid, a possible missetting of the reference ISFET electrode component 2 is corrected, while in the second calibrating step, a possible mis-setting of the measuring ISFET electrode component 1
is corrected.

It is also possible to perform an _in vivo_ calibration of the ion concentration measurement system according to this invention of which the reference FET electrode
is not provided with a (on-chip) Ag/AgCl electrode in
following manner. First blood is admitted into the lumen 8
and into contact with the reference ISFET electrode component 2, which for example is a pH-FET, and the output signal
$V_O$ of the differential circuit is noted. If the $V_O$ has a
value of other than zero mVolts, then the setting of the
differential circuit is adjusted until the magnitude of the
output signal $V_O$ of the differential circuit is zero mVolts.
In a subsequent, second step, a rinsing fluid with a pH
different from that of the original rinsing fluid is admitted
into the lumen 8 and in contact with the reference ISFET
electrode component 2, and the output signal $V_O$ of the
differential circuit is noted.
The output voltage $V_O$ now should be in accordance with the
pH difference between the pH value of this rinsing solution
and that of the originally used rinsing solution. If the $V_O$
displays another value setting of the circuit is adjusted
until the magnitude of the output voltage shows the proper
value. Before employing the ion concentration measurement
system to accomplish actual measurement or monitoring, the
calibration rinsing solution in lumen 8 can be expelled
from the lumen 8 by means of the original rinsing solution, after which the ion concentration measurement is suitably calibrated and ready for use.

The first calibrating step in which the reference ISFET electrode is exposed to blood a possible off-set mis-setting between ISFET electrode 1 and 2 is corrected, while in the second step a possible change in pH-sensitivity of the system is corrected.

If the chip of the reference ISFET electrode component also includes a temperature sensor _in vivo_ calibration is possible as discussed above. Additionally, such is accomplished without requiring special catheter construction and without the necessity of having to take blood samples by which by the calibration must be performed.

Although it is desirable to have the reference ISFET electrode component 2 mounted at a location that is generally close to that of the measuring ISFET electrode component 1, such as the construction shown in Figs. 1 and 2, it is possible to mount the reference ISFET electrode component 2 at a more remote location, such as within the bifurcation 3, within the liquid connector or tube 4, or even within the external rinsing liquid system to which the tubing 15 is attached.

It will be understood that the embodiments of the present invention which have been described are illustrative of some of the applications of the principles of the present invention. Numerous modifications may be made by those skilled in the art without departing from the true spirit and scope of the invention.

## Claims

1. An ion concentration measurement system suitable for _in vivo_ measurement of the concentration of an ion within a liquid, the system comprising:

a measuring ISFET electrode component having means for electrical communication with a voltage source for applying drain source voltage within the measuring ISFET electrode component and for applying gate voltage, said measuring ISFET electrode component being sensitive to a preselected ion;

a reference ISFET electrode component that is sensitive to the same preselected ion with respect to which the measuring ISFET electrode component is sensitive, and said reference ISFET electrode component has means for electrically communicating with a voltage source for applying drain source voltage within the reference ISFET electrode component and for applying gate voltage;

means for contacting said reference ISFET electrode component with a rinsing liquid of a known composition and ion concentration value; and

differential circuit means that electrically connects said measuring ISFET electrode component with said reference ISFET electrode component.

2. The ion concentration measurement system according to claim 1, further comprising an ion-selective electrode that is electrically combined with said reference ISFET electrode component.

3. The ion concentration measurement system according to claim 2, wherein said ion-selective electrode is an Ag/AgCl electrode.

4. The ion concentration measurement system according to claim 1, wherein each of said measuring and reference ISFET electrode components is a pH-FET.

5. The ion concentration measurement system according to claim 1, wherein said measuring ISFET electrode component is mounted within a top portion of a catheter.

6. The ion concentration measurement system according to claim 1, wherein said measuring ISFET electrode component is mounted in a portion of a catheter, and said catheter includes a metal electrode ground.

7. The ion concentration measurement system according to claim 1, wherein said means for contacting said reference ISFET electrode component with rinsing liquid includes a lumen of a catheter, through which lumen the rinsing liquid can flow.

8. The ion concentration measurement system according to claim 7, wherein said lumen of said means for contacting the reference ISFET electrode component with rinsing liquid includes an opening through the wall of the catheter.

9. The ion concentration measurement system according to claim 8, wherein said reference ISFET electrode component is mounted within said lumen of the means for contacting the reference ISFET electrode component with rinsing liquid.

10. The ion concentration measurement system according to claim 1, wherein the measuring ISFET electrode component is mounted within a top portion of a catheter, said catheter having at least two lumens, one lumen enclosing electrical conductors for said measuring ISFET electrode component, and the other lumen being included within said means for contacting said reference ISFET electrode component with rinsing liquid.

11. The ion concentration measurement system according to claim 10, wherein said reference ISFET electrode component is mounted within said lumen of the rinsing liquid contacting means.

12. The ion concentration measurement system according to claim 10, wherein said reference ISFET electrode component is mounted in generally close proximity to said measuring ISFET electrode component.

13. The ion concentration measurement system according to claim 1, wherein said differential circuit means includes a bridge circuit.

FIG.1

0155725

FIG.2

FIG.3

FIG.4

0155725

European Patent Office

**0155725**

. Application number

EP  85 20 0263

# EUROPEAN SEARCH REPORT

## DOCUMENTS CONSIDERED TO BE RELEVANT

| Category | Citation of document with indication. where appropriate, of relevant passages | Relevant to claim | CLASSIFICATION OF THE APPLICATION (Int. Cl 4) |
|---|---|---|---|
| D,Y | FR-A-2 304 083 (UNIVERSITY OF UTAH) <br> * Page 16, line 5 page 18, line 2; figures 4A,4B * & US - A - 4 020 830 (Cat. A) | 1 | G 01 N  27/30 |
| A | | 3,5 | |
| | --- | | |
| Y | IBM TECHNICAL DISCLOSURE BULLETIN, vol. 19, no. 4, September 1976, pages 1293-1294, New York, US; G.BARTHOLD et al.: "Single-rod measuring element for pH compensated redox potential measurement" <br> * Page 1292 * | 1 | |
| | --- | | |
| A | GB-A-2 030 358 (T.MUKAIBO) <br> * First page * | 1 | TECHNICAL FIELDS SEARCHED (Int. Cl.4) <br><br> G 01 N |
| | --- | | |
| A | PATENTS ABSTRACTS OF JAPAN, vol. 6, no. 30(P-103)(908), 23rd February 1982; & JP - A - 56 150 345 (KAZUMUKI YANAGISAWA) 20-11-1981 <br> * The whole document * | 1 | |
| | --- | | |
| A | GB-A-2 017 400 (M.YANO) <br> * First page * | 1 | |
| | ---                    -/- | | |

The present search report has been drawn up for all claims

| Place of search | Date of completion of the search | Examiner |
|---|---|---|
| THE HAGUE | 30-05-1985 | DUCHATELLIER M.A. |

# European Patent Office

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | DE-A-3 144 459 (T.AOMI)<br>* First page * | 1 | |

----

TECHNICAL FIELDS SEARCHED (Int. Cl.4)

The present search report has been drawn up for all claims

| Place of search | Date of completion of the search | Examiner |
|---|---|---|
| THE HAGUE | 30-05-1985 | DUCHATELLIER M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82